# DEMANDE DE BREVET EUROPEEN

(11) **EP 0 655 228 A1**
(43) Date de publication de la demande: **31.05.1995**
(21) Numéro de dépôt: 94402666.5
(22) Date de dépôt: 22.11.1994
(51) Int. Cl.: A61F 2/02

(54) **Filtre sanguin endovasculaire à deux séries d'éléments en forme de pétales**

(30) Priorité: 29.11.1993 FR 9314248
(71) Demandeur: B. BRAUN CELSA, Société Anonyme, F-86360 Chasseneuil du Poitou (FR)
(72) Inventeur: Chevillon, Gérard, F-92120 Montrouge (FR); Roussigne, Maurice, F-86000 Poitiers (FR); Nadal, Guy, F-86000 Poitiers (FR)
(74) Mandataire: Lerner, François

(57) **Abrégé**

Il s'agit d'un filtre sanguin destiné à être implanté sur le système circulatoire pour la retenue de caillots de sang.

Ce filtre présente un axe (6) et comporte des pattes élastiques (4, 14) en forme de boucles de fil(s) souple(s) définissant chacune une surface, ces pattes issues d'une tête (2) étant expansibles radialement à l'axe. Ce filtre se caractérise en ce que ses pattes définissent une première et une seconde séries de pattes, la seconde série coiffant la première, la surface définie par l'une quelconque des boucles (14) de la seconde série de pattes recouvrant, en tout ou partie, au moins la surface définie par une boucle (4) sous-jacente de la première série qu'elle coiffe.

L'invention s'applique tout particulièrement aux filtres temporaires en facilitant leur ouverture et leur retrait.

## Description

L'invention a pour objet un filtre sanguin perfectionné destiné à être placé dans un vaisseau du système circulatoire pour la retenue de caillots de sang.

Bien entendu, il existe déjà sur le marché différents types de filtres sanguins, implantables généralement à l'intérieur de la veine cave arrivant au coeur pour arrêter, avant leur entrée dans le coeur, les éventuels caillots de sang pouvant se former et risquant d'entraîner des embolies.

Parmi ces filtres, on en connaît qui sont fabriqués à partir de fils métalliques bouclés assemblés entre eux par soudage ou sertissage pour constituer des pattes en "pétales", ces pattes en boucles formant, en position implantée du filtre, un petit panier tronconique disposé dans le vaisseau. Ces filtres ont notamment l'avantage d'être peu encombrants en position repliée et peuvent donc être facilement implantés. Un exemple peut être trouvé au brevet US 5133733 où les pattes en fils bouclés sont associées à des pattes rectilignes terminées par un crochet permettant au filtre de s'ancrer à la paroi du vaisseau, en constituant alors ce que l'on dénomme habituellement un filtre "définitif" (non retirable aisément). On notera qu'au demeurant ces pattes en fil(s) bouclé(s) sont également connues pour être utilisées sur des filtres dits "temporaires", c'est-à-dire dépourvus de crochets (ou équivalents) d'ancrage au vaisseau, afin de permettre leur retrait une fois que leur temps d'intervention est terminé. La demande FR 9209957 du 12 Août 1992 donne un exemple d'un tel filtre.

Dans la pratique, on a toutefois constaté que ces filtres "en pétales" posaient souvent des problèmes liés à leur structure dans la mesure où la souplesse des fils augmente les risques d'emmêlement des pattes au moment de la pose dans le vaisseau, voire au moment du retrait pour les filtres temporaires.

La présente invention a pour objet de résoudre ces inconvénients en proposant un filtre perfectionné dont notamment la disposition relative particulière des pattes garantit une ouverture et un positionnement favorables du filtre dans le vaisseau, en limitant grandement les risques d'emmêlement, tout en favorisant le centrage du filtre.

Le filtre conforme à l'invention est tel que les pattes en boucles de fil(s) souple(s), définissant chacune le contour d'une surface, dont il est équipé sont réparties autour de la tête en une première et une seconde séries de pattes, la seconde série de pattes coiffant la première, la surface définie par l'une quelconque des boucles de la seconde série de pattes recouvrant, en tout ou partie, la surface définie par une boucle sous-jacente de la première série.

Dans ce cadre général des filtres "en pétales", l'invention s'applique tout particulièrement aux filtres "temporaires" dépourvus de moyens d'ancrage à la paroi du vaisseau et qui peuvent donc être implantés de manière amovible, l'invention permettant leur retrait aisé. En effet, la prévision sur ces filtres temporaires de deux séries superposées de boucles en fil, avec recouvrement au moins partiel comme indiqué ci-avant, permet au moment du retrait du filtre de le replier aisément sous un faible volume, les pattes se positionnant automatiquement les unes par rapport aux autres de manière ordonnée. Le filtre peut donc notamment être facilement et rapidement (re)introduit dans la gaine tubulaire utilisée couramment pour son retrait.

Selon une caractérisque de construction préférée de l'invention, les pattes seront toutes assemblées à une extrémité, sensiblement en une même zone de réunion, dans une tête commune et unique, en forme de calotte.

Avantageusement, chaque patte d'une des séries sera disposée radialement entre deux pattes adjacentes de l'autre série, la surface définie par l'une quelconque des boucles de la seconde série de pattes recouvrant, en tout ou partie, deux surfaces définies respectivement par deux boucles adjacentes de la première série.

Selon une caractérisque d'un mode de réalisation préféré de l'invention, les pattes de la seconde série seront à la fois plus courtes et plus renflées que celles de la première série ; ces première et seconde séries étant déployables respectivement suivant une première et une seconde corolles superposées.

L'invention et sa mise en oeuvre apparaîtront plus clairement de la description qui va suivre faite en référence aux dessins annexés donnés uniquement à titre d'illustration. Dans ces dessins :
- la figure 1 montre schématiquement, en perspective, le filtre de l'invention mis en place dans un vaisseau sanguin ;
- la figure 2 montre une vue agrandie du filtre de la figure 1, faite selon la flèche II sur la figure 1 ;
- la figure 3 est une vue agrandie d'un premier type de patte du filtre de la figure 1 ;
- la figure 4 est une vue agrandie d'un second type de patte du filtre de la figure 1 ;
- la figure 5 est une vue partielle agrandie d'un groupe de trois pattes du filtre de la figure 1 ;
- la figure 6 est une vue de côté dans le sens de la flèche VI de la figure 3 ;
- la figure 7 est une vue de côté dans le sens de la flèche VII de la figure 4 ;
- la figure 8 est une vue partielle, à plus grande échelle, avec arrachements, d'une possible réalisation de la tête du filtre de la figure 1 ;
- La figure 9 est une vue en coupe faite selon les flèches IX de la figure 8 ;
- la figure 10 représente, en position repliée du filtre dans son dispositif d'implantation, le groupe de pattes représenté à la figure 5.

Comme précisé précédemment, l'un des intérêts de l'invention étant son application aux filtres "temporaires", on ne décrira ci-après qu'un tel filtre même s'il doit être clair que l'invention peut s'appliquer à des filtres "définitifs", sur lesquels pourront alors être rapportés de manière connue en soi des moyens d'accrochage (se reporter par exemple au brevet US-A-5133733).

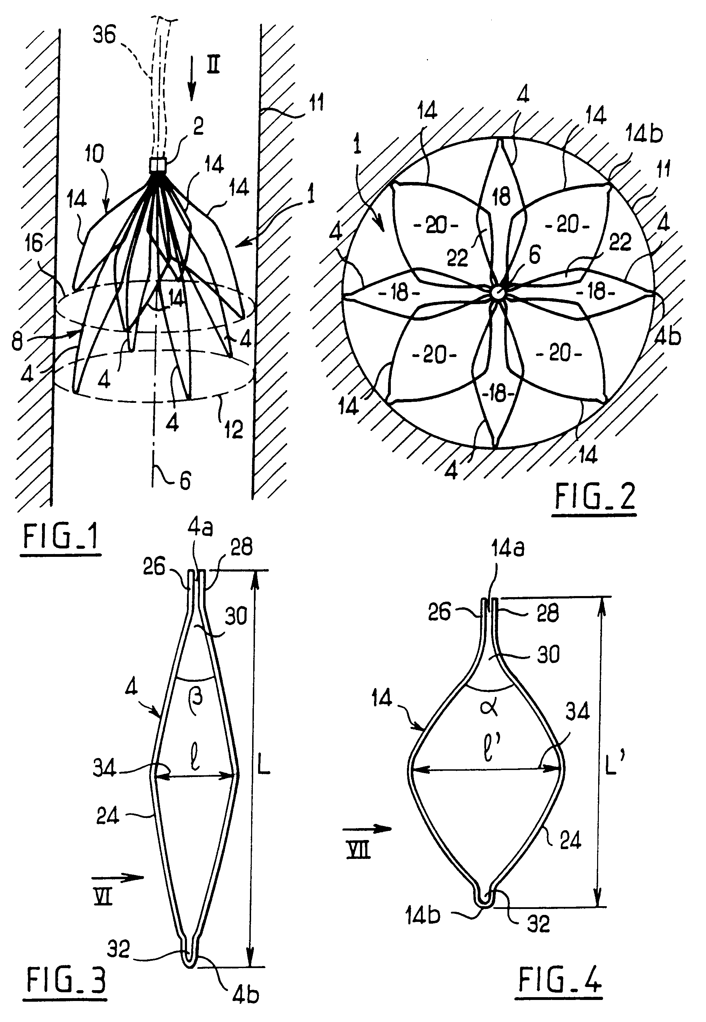

En se reportant aux figures 1 et 2, on voit représenté un filtre conforme à l'invention, référencé dans son ensemble en 1, qui a été placé dans une veine 11. Ce filtre est constitué par des pattes élastiques, par exemple au nombre de huit, rassemblées entre elles dans une tête 2.

Ce filtre 1 comprend deux types différents de pattes, plus précisément une première série de quatre pattes 4 coiffées par une seconde série de quatre autres pattes 14.

Les pattes 4 et 14 présentent une extrémité de réunion (ou proximale) 4a, 14a et une extrémité distale, libre, 4b, 14b. Elles sont déployables ou auto-expansibles radialement à l'axe général 6 du filtre, pour que leur extrémité libre 4b, 14b viennent au contact de la paroi du vaisseau lorsque le filtre y est implanté.

Liées à la tête, ou calotte de réunion 2, autour de laquelle elles sont radialement sensiblement régulièrement réparties, les pattes 4, 14 définissent respectivement une première corolle 8 et une seconde corolle 10 surplombant la première, toutes deux sensiblement coniques d'axe 6.

Avantageusement, les pattes 14 sont plus courtes que les pattes 4. Ainsi, l'ouverture marquée en pointillés 12 de la corolle 8, est située à un niveau inférieur à celle marquée en pointillés 16 de la corolle 10. Cet étagement, parallèlement à l'axe 6, des zones de contact des pattes avec la paroi du vaisseau donne une plus grande stabilité au filtre. De plus, cette prévision de pattes courtes et longues permet de replier le filtre sous un plus faible volume, facilitant encore son implantation ou son retrait dans le vaisseau.

Comme il apparaît clairement à la figure 2, les pattes sont réalisées à partir d'au moins un fil souple replié sur lui-même sensiblement en forme de boucle, dont le contour définit une surface à aspect de pétale. Une boucle de la première série et une boucle de la deuxième série délimitent respectivement des surfaces référencées 18 et 20 qui seront avantageusement planes ou légèrement bombées à convexité dirigée vers l'extérieur (paroi du vaisseau). Les pattes d'une même série sont de préférence écartées les unes des autres sans contact et chaque patte d'une des séries est située entre deux pattes adjacentes de l'autre série. Les pattes des deux séries étant ainsi intercalées, chaque surface 20 couvre, au moins en projection et partiellement, une surface 18 et de préférence, comme représenté ici, deux surfaces 18 qui lui sont directement adjacentes.

Plus particulièrement sur les figures 3 et 4, on remarquera que chacune des pattes 4 et 14 est ici réalisée à partir d'un fil unique 24 recourbé sur lui-même de manière que ses extrémités libres opposées 26, 28 soient rassemblées, prêtes à être fixées dans la tête 2.

En tant que fil(s), on pourra utiliser en particulier des fils métalliques, de section circulaire de quelques dixièmes de millimètres, tels qu'en acier inoxydable de qualité appropriée, par exemple connu sous leur nom de marque déposée "Phynox".

Les pattes 4 et 14 présentent, de préférence, une partie étroite 30 vers leur extrémité de réunion et une partie extrême étranglée 32, vers leur extrémité libre opposée, l'étranglement 32 étant favorable à la rigidité relative des pattes. Toutes les pattes 4 de la première série et toutes celles 14 de la seconde série présentent en position déployée respectivement une longueur L et une longueur L' (L ≧ L') qui s'étendent entre leurs extrémités proximales et distales. Elles comportent également, de préférence sensiblement à la moitié de leur longueur respective, une partie ventrue 34. Les pattes courtes 14 étant avantageusement plus renflées que les pattes longues, cette partie 34 aura, pour la patte 14, une largeur l' supérieure ou égale à celle l de la patte 4. On notera que, de manière préférée, les pattes auront toutes une forme de losange.

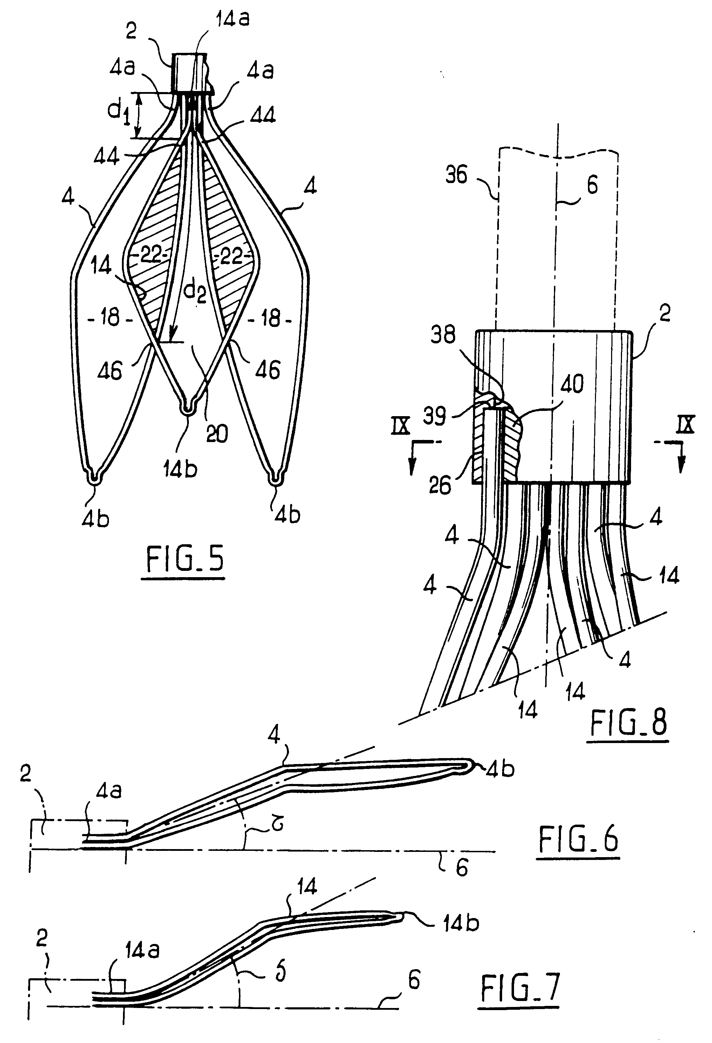

A la figure 5, on a représenté plus précisément, en hachuré, les zones de recouvrement 22 des surfaces 20 et 18. Le filtre est conformé de telle sorte que ces zones soient relativement importantes, notamment dans sa position repliée. De cette façon, ces zones assurent un recouvrement ordonné et stable des pattes de la première sèrie par celles de la seconde série. On remarquera ici deux points de "recouvrement" ou de "croisement" 44, 46 entre le fil d'une boucle supérieure 14 et le fil d'une boucle inférieure adjacente 4. Le point 44 est situé vers la zone rétrécie 30 et à proximité immédiate des extrémités 4a, 14a, c'est-à-dire à une distance d₁ depuis l'extrémité de réunion des pattes comprise entre quasiment cette extrémité et environ 1/4 de la longueur L. Le point 46 est situé vers la partie ventrue 34, de préférence après cette partie lorsque l'on se déplace en direction de l'extrémité distale des pattes, c'est-à-dire à une distance d₂ depuis l'extrémité de réunion des pattes comprise entre environ 1/2 et 3/4 de la longueur L des pattes 4.

Sur les figures 6 et 7, les pattes 4 et 14 ont respectivement été représentées en vue de côté, avec ici le profil courbe qu'elles prendront naturellement de manière que le filtre 1, dans un état non contraint, ait tendance à se déployer radialement. Ces figures montrent en outre que, la patte 14 étant plus courte, elle est, vers son extrémité proximale, naturellement plus écartée de l'axe 6 (angle δ) que la patte 4 (angle τ).

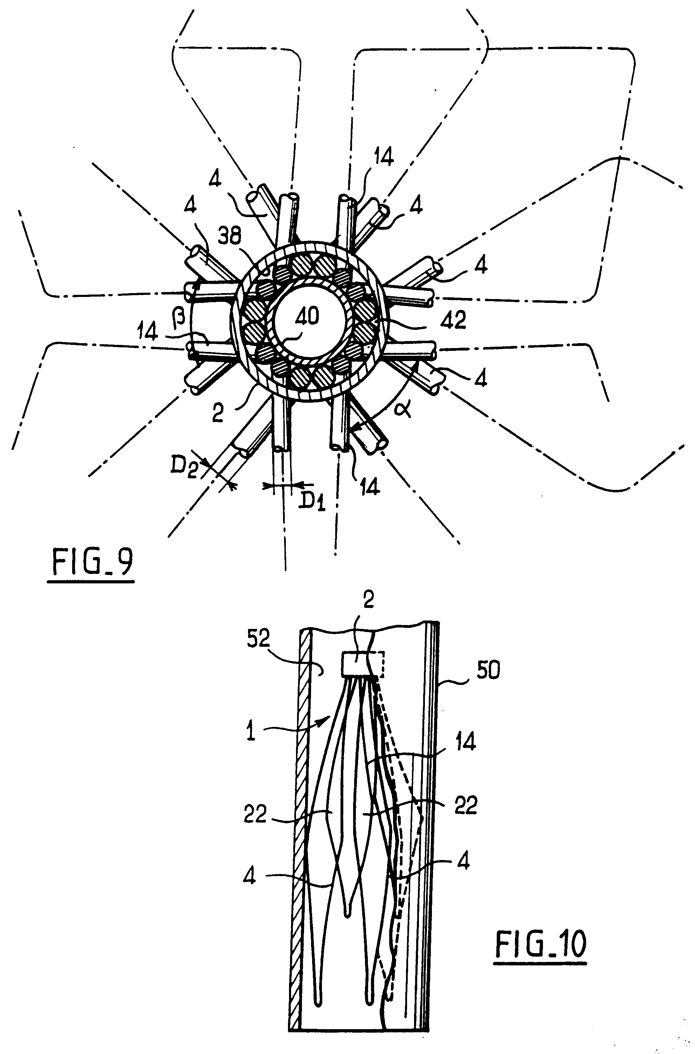

Les figures 8 et 9 montrent une manière possible de fixer les pattes dans la tête ou calotte 2, laquelle est ici raccordable axialement, à l'opposé, (par sertissage) à un élément de maintien du filtre, schématisé en pointillés 36 et pouvant être constitué d'un câble, tube ou tige souple, par exemple en matière plastique biocompatible, destiné à permettre la manoeuvre du filtre depuis l'extérieur du corps du patient (à la manière de ce qui est prévu pour quasiment tous les filtres temporaires actuels).

Tel qu'illustré, la tête 2, sensiblement cylindrique, présente, en direction du filtre, un alésage intérieur 38 (formant un décrochement ou épaulement intérieur 39) dans lequel sont disposées les extrémités libres opposées 26, 28 des fils. Ces fils, en appui contre l'épaulement 39, seront bloqués grâce à une pièce centrale 40, sensiblement cylindrique, délimitant un espace annulaire périphérique 42 de logement des fils qui y sont régulièrement répartis.

Les deux extrémités libres opposées d'un fil sont disposées côte à côte et sont accolées de part et d'autre aux extrémités libres opposées du fil d'une boucle d'une série différente.

Avantageusement, les pattes courtes 14 sont constituées d'un fil souple de diamètre D₁ inférieur au diamètre D₂ des pattes longues, cette différence de diamètre combinée à la différence de longueur des pattes donnant à toutes les boucles une rigidité sensiblement égale. Par exemple, D₁ pourra être égal à environ 0,25 mm et D₂ à environ 0,30 mm.

En outre, on remarquera qu'une patte 14 de la seconde sérié présente, en position déployée et vers son extrémité de réunion à la tête, un angle α d'ouverture de la boucle qu'elle forme supérieur ou égal à l'angle β d'ouverture d'une boucle que présente une patte 4 vers son extrémité proximale 4a, en position déployée. Cet angle α sera de préférence compris entre 30° et 50°, l'angle β étant de préférence compris entre 15° et 25° (on notera que sur les figures les angles α et β apparaissent plus importants pour des raisons de clarté notamment).

Le filtre selon l'invention pourra être mis en place de manière connue en soi, par dénudation ou par voie percutanée (par exemple par la technique dite de "SELDINGER") en recourant à un dispositif d'implantation comprenant une gaine tubulaire souple pouvant renfermer intérieurement, le filtre en position radialement contrainte.

A la figure 10, on a représenté le filtre 1 disposé en position repliée dans le passage intérieur 52 d'une telle gaine 50. Dans cette position où les pattes 14 sont en contact et recouvrent les pattes 4 le long de l'axe 6, il n'y a pratiquement pas de risque d'emmêlement de ces pattes.

Au moment du "lâcher" du filtre dans la veine, on observe également une expansion ordonnée des deux séries de pattes jusqu'à ce qu'elles viennent au contact de la paroi ; ces deux séries étant, de préférence, en contact entre elles durant une partie au moins de leur expansion radiale, pour favoriser leur guidage en ouverture, les angles d'écartement τ, δ donnés naturellement aux pattes 4, 14 respectivement par rapport à l'axe du filtre, étant adaptés en conséquence (τ ≦ δ), avec τ compris de préférence entre 10° et 30° et δ compris de préférence entre 15° et 40°.

Bien entendu, l'invention n'est nullement limitée au mode de réalisation décrit. Ainsi, on pourrait envisager que toutes les pattes aient sensiblement la même longueur, les corolles 8 et 10 étant alors en contact et en appui l'une sur l'autre lors de l'ouverture du filtre et dans sa position implantée. On pourrait également envisager d'employer un seul fil pour constituer toutes les pattes d'une même série, voire des deux.

## Revendications

1. Filtre sanguin destiné à être implanté dans un vaisseau en particulier pour la retenue de caillots de sang, ce filtre (1) présentant un axe (6) et comportant une tête (2) de laquelle sont issues des pattes élastiques (4, 14) expansibles sensiblement radialement à l'axe du filtre et en forme de boucles de fil(s) souple(s) (24) définissant chacune le contour d'une surface (18, 20), le filtre se caractérisant en ce que ses pattes sont réparties autour de la tête en une première et une seconde séries de pattes, la seconde série coiffant la première, la surface (20) définie par l'une quelconque des boucles (14) de la seconde série de pattes recouvrant, en tout ou partie, au moins la surface (18) définie par une boucle (4) sous-jacente de la première série qu'elle coiffe.

2. Filtre sanguin destiné à être implanté de manière amovible dans un vaisseau en particulier pour la retenue de caillots de sang, ce filtre, dépourvu de moyens d'ancrage à la paroi du vaisseau, présentant un axe (6) et comportant une tête (2) de laquelle sont issues des pattes élastiques (4, 14) expansibles sensiblement radialement à l'axe du filtre et en forme de boucles de fil(s) souple(s) (24) définissant chacune le contour d'une surface (18, 20), le filtre se caractérisant en ce que ses pattes sont réparties autour de la tête en une première et une seconde séries de pattes, la seconde série coiffant la première, la surface (20) définie par l'une quelconque des boucles (14) de la seconde série de pattes recouvrant, en tout ou partie, au moins la surface (18) définie par une boucle (4) sous-jacente de la première série qu'elle coiffe.

3. Filtre sanguin selon la revendication 1 ou la revendication 2, caractérisé en ce que toutes les pattes qui définissent, en position expansée, une corolle (8, 10) sensiblement conique, sont rassemblées à une extrémité (4a, 14a), sensiblement en une même zone de réunion dans une tête commune (2) définissant une calotte et présentent une extrémité libre (4 b, 14 b) à l'opposé de cette dite zone de réunion.

4. Filtre sanguin selon l'une quelconque des revendications 1 à 3, caractérisé en ce que chaque patte d'une des séries est disposée radialement entre deux pattes adjacentes de l'autre série, ladite surface (20) définie par l'une quelconque des boucles (14) de la seconde série recouvrant, en tout ou partie, deux surfaces (18) définies chacune par une boucle (4) de la première série.

5. Filtre sanguin selon l'une quelconque des revendications 1 à 4, caractérisé en ce que les pattes (4) de la première série sont plus longues que les pattes (14) de la seconde série.

6. Filtre sanguin selon l'une quelconque des revendications 1 à 5, caractérisé en ce que les pattes (4) de la première série sont moins renflées que les pattes (14) de la seconde série.

7. Filtre sanguin selon l'une quelconque des revendications 1 à 6, caractérisé en ce que la seconde série de pattes (14) coiffe la première série de pattes (4) de telle sorte que ces deux séries soient en contact entre elles en position repliée du filtre et durant une partie au moins de l'expansion radiale de l'ensemble des pattes.

8. Filtre sanguin selon l'une des revendications précédentes, caractérisé en ce que les pattes (4, 14) sont façonnées sensiblement en forme de losange.

9. Filtre sanguin selon l'une des revendications précédentes, caractérisé en ce que toutes les pattes d'une même série (4, 14) présentent une longueur déterminée (L, L') depuis leur extrémité (4a, 14a) de réunion à la tête (2) du filtre jusqu'à leur extrémité libre opposée (4b, 14b), une boucle de la deuxième série de pattes présentant deux points de recouvrement (44, 46) d'au moins une boucle de la première série, ces points étant situés, l'un à proximité de l'extrémité de réunion des pattes, l'autre à environ 1/2 à 3/4 de la longueur desdites pattes de la première série depuis leur extrémité de réunion.

10. Filtre sanguin selon l'une quelconque des revendications précédentes, caractérisé en ce que chaque patte de la deuxième série (14) présente, du côté de son extrémité de réunion à la tête, un angle (α) d'ouverture de la boucle qu'elle forme supérieur ou égal à l'angle (β) d'ouverture de la boucle d'une patte de la première série, vers l'extrémité de réunion de celle-ci à ladite tête du filtre.

11. Filtre sanguin selon l'une quelconque des revendications précédentes, caractérisé en ce que chaque patte de la deuxième série (14) s'écarte naturellement de l'axe du filtre, du côté de son extrémité de réunion à la tête, d'un angle (δ) supérieur ou égal à l'angle (τ) d'écartement d'une patte de la première série, vers son extrémité de réunion à la tête, par rapport à l'axe du filtre.
